# EUROPÄISCHE PATENTSCHRIFT

(11) **EP 1 436 403 B1**
(45) Veröffentlichungstag und Bekanntmachung des Hinweises auf die Patenterteilung: **02.03.2011**
(21) Anmeldenummer: 02785218.5
(22) Anmeldetag: 15.10.2002
(51) Int. Cl.: C12N 15/86, C12N 15/85, A61K 48/00, C07K 14/47, C12N 15/12

(54) **VERWENDUNG DES ADENOVIRALEN E2-LATE-PROMOTORS**
USE OF THE ADENOVIRAL E2 LATE PROMOTER
UTILISATION DU PROMOTEUR TARDIF E2 ADENOVIRAL

(30) Priorität: 16.10.2001 DE 10150984
(43) Veröffentlichungstag der Anmeldung: 14.07.2004
(73) Patentinhaber: Technische Universität München, 80333 München (DE)
(72) Erfinder: HOLM, Per, Sonne, 82256 Fürstenfeldbruck (DE)
(74) Vertreter: Bohmann, Armin K.
(86) Internationale Anmeldenummer: PCT/EP2002/011527
(87) Internationale Veröffentlichungsnummer: WO 2003/033692

(56) Entgegenhaltungen:
- WO-A-01/02556
- WO-A-01/70951
- WO-A-97/16547
- BHAT, G. ET AL: "In vivo identification of multiple promoter domains of adenovirus EIIA-late promoter" EMBO JOURNAL, Bd. 6, Nr. 7, 1987, Seiten 2045-2052, XP001148840 EYNSHAM, OXFORD GB
- SWAMINATHAN S ET AL: "REGULATION OF ADENOVIRUS E2 TRANSCRIPTION UNIT" CURRENT TOPICS IN MICROBIOLOGY AND IMMUNOLOGY, SPRINGER, BERLIN,, DE, Bd. 199, Nr. PART 3, 1995, Seiten 177-194, XP001015599 ISSN: 0070-217X in der Anmeldung erwähnt
- LADOMERY M ET AL: "A ROLE FOR Y-BOX PROTEINS IN CELL PROLIFERATION" BIOASSAYS, CAMBRIDGE, GB, Bd. 17, Nr. 1, 1995, Seiten 9-11, XP000978744 ISSN: 0265-9247 in der Anmeldung erwähnt
- KOIKE K ET AL: "Nuclear translocation of the Y-box binding protein by ultraviolet irradiation" FEBS LETTERS, ELSEVIER SCIENCE PUBLISHERS, AMSTERDAM, NL, Bd. 417, Nr. 3, 17. November 1997 (1997-11-17), Seiten 390-394, XP002177054 ISSN: 0014-5793
- OHGA TAKEFUMI ET AL: "Role of the human Y box-binding protein YB-1 in cellular sensitivity to the DNA damaging agents cisplatin, mitomycin C, and ultraviolet light" CANCER RESEARCH, AMERICAN ASSOCIATION FOR CANCER RESEARCH, BALTIMORE, MD, US, Bd. 56, Nr. 18, 1996, Seiten 4224-4228, XP002157301 ISSN: 0008-5472
- OHGA TAKEFUMI ET AL: "Direct involvement of the Y-box binding protein YB-1 in genotoxic stress-induced activation of the human multidrug resistance 1 gene" JOURNAL OF BIOLOGICAL CHEMISTRY, AMERICAN SOCIETY OF BIOLOGICAL CHEMISTS, BALTIMORE, MD, US, Bd. 273, Nr. 11, 13. März 1998 (1998-03-13), Seiten 5997-6000, XP002157299 ISSN: 0021-9258
- HOLM PER S; ET AL.: "YB-1 relocates to the nucleus in adenovirus infected cells and facilitates viral replication E2 gene expression through the E2--- late promoter" JOURNAL OF BIOLOGICAL CHEMISTRY , 22. März 2002 (2002-03-22), XP002237181

### SEQUENZPROTOKOLL

<110> Dr. Holm, Per Sonne
<120> Verwendung des adenoviralen E2-late-Promotors
<130> H10005 PCT
<140>
   <141>
<160> 2
   <170> PatentIn Ver. 2.1
<210> 1
   <211> 74
   <212> DNA
   <213> Adenovirus
<220>
   <223> Fragment des E2-late-Promotors
<400> 1 
atttgtacct gaggactacc acgcccacga gattaggttc tacgaagacc aatcccgccc
gccaaatgcg gagc 74
<210> 2
   <211> 41
   <212> DNA
   <213> Adenovirus
<220>
   <223> Fragment des E2-late-Promotors
<400> 2
   ccacgagatt aggttctacg aagaccaatc ccgcccgcca a 41

## Beschreibung

Die vorliegende Erfindung betrifft die Verwendung eines adenoviralen E2-late-Promotors oder eines funktional aktiven Fragmentes davon, ein Nukleinsäurekonstrukt umfassend einen durch YB-1 kontrollierten adenoviralen E2-late-Promotor oder eines funktional aktiven Fragmentes davon, einen dieses Nukleinsäurekonstrukt umfassenden Vektor und die Verwendung des Nukleinsäurekonstruktes.

Bei der Behandlung von Tumoren werden derzeit eine Vielzahl von Therapiekonzepten verfolgt. Neben der Verwendung chirurgischer Techniken stehen dabei die Chemotherapie und die Strahlentherpaie im Vordergrund. All diese Techniken sind jedoch für den Patienten mit nicht unerheblichen Nebenwirkungen verbunden.

Mit der Verwendung von replikationsselektiven onkolytischen Viren wurde eine neue technologische Plattform für die Behandlung von Tumoren geschaffen. Dabei wird eine selektive intratumorale Replikation eines viralen Agens herbeigeführt, die in der Folge zur Virusreplikation, Lyse der infizierten Tumorzelle und Verbreitung des Virus auf benachbarte Tumorzellen führt. Infolge der Beschränkung der Replikationsfähigkeit des. Virus auf Tumorzellen bleibt normales Gewebe von der Infektion und damit der Lyse durch den Virus verschont. Beispiele für derartige replikationsselektive onkolytische Viren sind Gen-attenuierte Adenovirus und Herpesviren (Martuza, R. et al. Science 252, 854-858 (1991); Fueyo, J et al. Oncogene 19, 2-12 (2000))

Adenoviren sind in der Technik bekannt. Dabei handelt es sich um dsDNA-Viren (Boulanger, P et al. (1991); Biochem. J. 275, 281-299). Die vollständige Nukleotidsequenz des adenoviralen Genoms ist bekannt und beschrieben in (Chroboczek, J. et al., Virology 1992, 186, 280-285). Ein für die Verwendung von Adenoviren besonders wichtiger Teil des Genoms sind die sogenannten frühen oder early Gene und deren Genprodukte, die als E1, E2, E3 und E4 bezeichnet werden. Dabei umfasst E1 zwei Genprodukten E1A und E1B, die Onkogene darstellen. Die insgesamt drei Genprodukte der Gruppe E2 sind zusammen mit den Genprodukten E3 und E4 an der Replikation beteiligt.

Ein Beispiel für einen onkolytischen Adenovirus ist dl 1520 (Onyx-015), der bereits erfolgreich in den klinischen Phasen I und II eingesetzt wurde (Khuri, F. et al. Nature Medicine 6, 879-885 (2000). Onyx-015 ist ein Adenovirus, bei dem das E1B 55-kDa-Gen deletiert ist. Das E1B-55kDa-Genprodukt ist beteiligt an der Inhibierung von p53, dem Transport viraler mRNA und dem Abschalten der Proteinsynthese der Wirtszelle. Die Inhibierung von p53 erfolgt dabei durch Ausbilden eines Komplexes aus p53 und dem adenoviral codierten E1B-55 kDa-Protein. Von p53, codiert von TP53, geht ein komplexer regulatorischer Mechanismus aus (Zambetti, G.P. et al., FASEB J, 7, 855-865), der u.a. auch dazu führt, dass eine effiziente Replikation von Viren wie Adenoviren in der Zelle unterdrückt wird. Das Gen TP 53 ist in etwa 50 % aller menschlichen Tumoren deletiert oder mutiert mit der Folge, dass es zu keiner - erwünschten - Apoptose infolge einer Chemotherapie oder einer Bestrahlungstherapie kommt und damit der Erfolg dieser Tumorbehandlungen im Normalfall unterbleibt.

DNA-Tumorviren, wie Adenoviren, treiben die infizierten Zellen in die S-Phase des Zellzyklus, um die virale DNA-Replikation zu erleichtern. Onyx-015 exprimiert das E1B-55 kDa Protein nicht und repliziert selektiv in Tumorzellen gegenüber normalen Zellen. Darüberhinaus besteht eine weitere Selektivität dahingehend, dass solche Tumoren infolge der viralen Lyse der Tumorzellen eine vergleichsweise stärkere Nekrose erfahren, die p53-defizient sind, als jene, die den p53-Wildtyp aufweisen (Khuri et al., aaO). Trotz der grundsätzlichen Wirksamkeit von Onyx-015 bei der viral induzierten Onkolyse im Falle von p53-defizienten Tumoren ist die Erfolgsrate von 15 % der behandelten Tumore sehr gering.

Ries et al. (Ries, S.J. et al. Nature Medicine 6, 1128 - 1132 (2000)) haben eine prinzipielle Möglichkeit aufgezeigt, wie Onyx-015 auch bei Tumoren mit p53-Wildtyp erfolgreich verwendet werden können. Dabei wird das Tumorsuppressor-Protein p14ARF nicht exprimiert. In Folge des Fehlens von p14ARF unterbleibt die normale Reaktion des p53-Systems auf eine virale Infektion und erlaubt damit die Replikation von Onyx-015 auch in diesen Tumoren. Eine Anwendung dieser Erkenntnis setzt jedoch voraus, dass in der Tumorzelle ein geeigneter genetischer Hintergrund existiert oder durch geeignete therapeutische Maßnahmen bereitgestellt wird. Im ersteren Falle würde sich die Anzahl der mittels Onxy-015 therapierbaren Tumoren weiter verringern, im zweiten Fall wäre eine aufwendige Änderung des genetischen Hintergrundes der Tumorzellen erforderlich.

Bhat G. et al., The EMBO Journal, Band 6, Nr. 7, Seiten 2045 - 2052, 1987, beschreiben die *in vivo* Identifizierung von multiplen Promotordomänen des adenoviralen EIIA-late-Promotors.

Swaminathan S und Thimmapaya, Current Topics in Microbiology and Immunology, Springer, Berlin, DE, Band 199, Teil 2, 1995, Seiten 177- 194 beschreiben die Steuerung der adenoviralen Transkriptionseinheit E2.

Der vorliegenden Erfindung liegt in einem Aspekt die Aufgabe zu Grunde einen Promotor bereitzustellen, der eine tumorspezifischen Expression von Nukleinsäuren erlaubt. In einem anderen Aspekt liegt der Erfindung die Aufgabe zugrunde, ein Mittel zur Therapie von YB-1 positiven Erkrankungen, insbesondere von Tumorerkrankungen bereitzustellen.

Erfindungsgemäß wird die Aufgabe in einem ersten Aspekt gelöst durch die Verwendung eines adenoviralen E2-late-Promotors oder eines funktional aktiven Fragments davon zur durch YB-1 kontrollierten Expression von Genen, die verschieden sind von den durch den E2-late-Promotor gesteuerten adenoviralen Genen oder Nukleinsäuren, zur Herstellung eines Medikamentes, wobei der Promotor und/oder das Promotor-Fragment eine Bindungsstelle für YB-1 aufweist.

Erfindungsgemäß wird die Aufgabe in einem zweiten Aspekt gelöst durch die in vitro-Verwendung eines adenoviralen E2-late-Promotors oder eines funktional aktiven Fragments davon zur durch YB-1 kontrollierten Expression von Genen, die verschieden sind von den durch den E2-late-Promotor gesteuerten adenoviralen Genen oder Nukleinsäuren, wobei der Promotor und/oder das Promotor-Fragment eine Bindungsstelle für YB-1 aufweist.

Erfindungsgemäß wird die Aufgabe in einem dritten Aspekt gelöst durch die Verwendung eines adenoviralen E2-late-Promotors oder eines funktional aktiven Fragments davon zur YB-1 kontrollierten Expression eines Transgens oder einer transgenen Nukleinsäure zur Herstellung eines Medikamentes, wobei der Promotor und/oder das Promotor-Fragment eine Bindungsstelle für YB-1 aufweist.

Erfindungsgemäß wird die Aufgabe in einem vierten Aspekt gelöst durch die in vitro-Verwendung eines adenoviralen E2-late-Promotors oder eines funktional aktiven Fragments davon zur YB-1 kontrollierten Expression eines Transgens oder einer transgenen Nukleinsäure, wobei der Promotor und/oder das Promotor-Fragment eine Bindungsstelle für YB-1 aufweist.

In einer Ausflihrungsform der erfindungsgemäßen Verwendungen ist vorgesehen, dass das Promotor-Fragment eine Sequenz gemäß SEQ. ID. No. 1 umfasst.

In einer alternativen Ausführungsform der erfindungsgemäßen Verwendungen ist vorgesehen, dass das Promotor-Fragment eine Sequenz gemäß SEQ. ID. No. 2 aufweist.

In einer Ausführungsform der erfindungsgemäßen Verwendungen ist vorgesehen, dass der Promotor und/oder das Promotor-Fragment eine Bindungsstelle für YB-1 aufweist.

In einer weiteren Ausführungsform der erfindungsgemäßen Verwendungen ist vorgesehen, dass der Promotor und/oder das Fragment mindestens ein Element aufweist, das ausgewählt ist aus der Gruppe, die die Y-Box, die TATA-Box und die SPI-Bindungsstelle umfasst.

In einer noch weiteren Ausführungsform der erfindungsgemäßen Verwendungen ist vorgesehen, dass der Promotor und/oder das Promotor-Fragment YB-1 gebunden aufweist.

In einer Ausführungsform der erfindungsgemäßen Verwendungen ist vorgesehen, dass das Transgen und/oder das von dem durch den E2-late-Promotor gesteuerte adenovirale Gen und/oder die von dem durch den E2-late-Promotor gesteuerte Nukleinsäure ausgewählt ist aus der Gruppe von Genen, die Apoptose-induzierenden Gene, Gene für Pro-Drug-Systeme und Gene für Protease-Inhibitoren umfasst.

In einer Ausführungsform der erfindungsgemäßen Verwendungen ist vorgesehen, dass das Transgen oder die von der durch den adenoviralen E2-late-Promotor gesteuerten adenoviralen Gene oder Nukleinsäure(n) ausgewählt ist/sind aus der Gruppe, die Antisense-Moleküle, Ribozyme Aptamere und siRNA umfasst.

In einem fünften Aspekt wird die Aufgabe erfindungsgemäß gelöst durch ein Nukleinsäurekonstruk zur Verwendung als Medikament umfassend einen durch YB-1 kontrollierten, adenoviralen E2-late-Promotor oder ein funktional aktivea Fragment davon und eine Nukleinsäure, wobei die Nukleinsäure ausgewählt ist aus der Gruppe, die Transgene, Gene und Nukleinsäuren, die jeweils verschieden sind von den durch einen E2-late-Promotor gesteuerten adenoviralen Nukleinsäure, umfasst.

In einer Ausführungsform ist vorgesehen, dass das Promotorfragment eine Nukleinsäuresequenz umfasst, die ausgewählt ist aus der Gruppe, die SEQ. ID. No. 1 und SEQ. ID. No. 2 umfasst.

In einem sechsten Aspekt wird die Aufgabe erfindungsgemäß gelöst durch einen Vektor umfassend ein erfindungsgemäßes Nukleinsäurekonstrukt.

In einem siebten Aspekt wird die Aufgabe erfindungsgemäß gelöst durch die Verwendung eines erfindungsgemäßen Nukleinsäurekonstruktes zur Herstellung eines Medikaments.

In einer Ausführungsform ist vorgesehen, dass das Promotorfragment eine Nukleinsäure umfasst, die ausgewählt ist aus der Gruppe, die SEQ. ID No. 1 und SEQ. ID No. 2 umfasst.

In einer Ausführungsform ist vorgesehen, dass das Medikament zur Behandlung von Tumoren verwendet wird.

In einer weiteren Ausührungsform ist vorgesehen, dass die Tumoren solche sind, die YB-1 im Kern aufweisen.

In einer noch weiteren Ausführungsform ist vorgesehen, dass die Tumoren solche sind, die YB-1 im Kern aufweisen, bevorzugter Weise bei Vorliegen eines Stressfaktors YB-1 im Zellkern aufweisen.

In einer Ausführungsform ist vorgesehen, dass der Stressfaktor ausgewählt ist aus der Gruppe, die Hyperthermie, UV-Exposition und Exposition gegenüber Zytostatika umfasst.

In einer noch weiteren Ausführungsform ist vorgesehen, dass das Medikament zusammen mit Zytostatika und/oder Hyperthermie verwendet wird.

Schließlich ist in einer noch weiteren Ausführungsform vorgesehen, dass der Tumor Tumorzellen mit Vielfachresistenzen aufweist.

Der vorliegenden Erfindung liegt die überraschende Erkenntnis zu Grunde, dass YB-1 im Kern an den adenoviralen E2-late-Promotor bindet und sich dieser Promotor auch hervorragend für die Expression von Nukleinsäuren eignet, die verschieden sind von jenen Nukleinsäuren, die in einem adenoviralen System, d.h. in einem natürlicherweise vorkommenden Adenovirus, durch den E2-late-Promotor gesteuert werden. Weiterhin wurde überraschenderweise festgestellt, dass der adenovirale E2-late-Promotor zum einen ein sehr starker Promotor und verglichen mit dem als Gold-Standard verwendeten CMV-Promotor nur unwesentlich schwächer ist bei einer praktisch nicht existierenden Hintergrundexpression für den Fall, dass der Promotor nicht aktiv ist.

Die erfindungsgemäße Verwendung des adenoviralen E2-late-Promotors wird insbesondere durch seine Regulierbarkeit durch YB-1 bestimmt, wobei YB-1 als positiver Effektor wirksam ist, d.h. der Promotor erst bei Anwesenheit von YB-1 im Kern aktiv ist. Insoweit ist der besagte adenovirale E2-late-Promotor hochselektiv regulierbar und somit in Systemen verwendbar, in denen YB-1 im Kern vorhanden ist und praktisch eine jegliche Expression der unter der Kontrolle des adenoviralen E2-late-Promotors stehenden Nukleinsäure verhindert für den Fall, dass YB-1 als Effektor bzw. Regulator im Kern nicht vorhanden ist.

YB-1 ist ein Vertreter der Y-Box-Proteinfamilie, die an das DNA-Sequenzmotiv Y-Box bindet. Das Y-Box-Motiv stellt ein transkriptionell regulatorisches Element dar, das sich in den Promotor- oder Enhancer-Regionen einer Anzahl unterschiedlicher Gene findet, die ein Rolle bei der Regulation der Zellproliferation spielen (Ladomery, M. et al., 1995; Bioassays 17: 9-11; Didier, D.K. et al., 1988, PNAS, 85, 7322-7326).

Die hierin gemachten Ausführungen gelten auch für Fragmente des besagten adenoviralen E2-late-Promotors, der hierin gelegentlich auch als E2-late-Promotor oder später E2-Promotor bezeichnet wird, und insbesondere für jene Promotor-Fragmente, die hierin offenbart und als SEQ. ID. NO. 1 und SEQ. ID. NO. 2 bezeichnet sind.

Die Nukleinsäuresequenz gemäß SEQ.ID.No. 1 lautet wie folgt:
5'-atttgtacctgaggactaccacgcccacgagattaggtt
ctacgaagaccaatcccgcccgccaaatgcggagc-3'

Die für die Bindung von YB-1 als relevant erachtete Y-Box (SAAT) ist in Fettdruck dargestellt.

Bei der Sequenz gemäß SEQ.ID.NO. 1 handelt es sich um den Bereich der Positionen - 22 bis - 96 des E2-late-Promotors

Die Nukleinsäuresequenz gemäß SEQ.ID.No. 2 lautet wie folgt:
5'- ccacgagattaggttctacgaagaccaatcccgcccgccaa-3'

Die Nukleinsäuresequenz gemäß SEQ.ID.No. 2 umfasst den Bereich der Positionen -47 bis - 87 des E2-late-Promotors.

Darüber hinaus ist es im Rahmen der vorliegenden Erfindung, dass ein jegliches Fragment oder Derivat des Promotors verwendet werden kann, solange dieses in der Lage ist, YB-1 zu binden und immer noch eine Promotoraktivität aufweist. Ohne im folgenden darauf festgelegt sein zu wollen, scheint die Bindung von YB-1 an der Y-Box zu erfolgen oder die Y-Box an der Ausbildung von Sekundärstrukturen beteiligt zu sein, weshalb das Vorhandensein dieser Box als für die Ausgestaltung des adenoviralen E2-late-Promotors und entsprechender erfindungsgemäß verwendeter Fragmente davon von Bedeutung ist.

Der E2-late-Promotor von Adenovirus ist beispielsweise beschrieben bei Swaminathan, S., and Thimmapaya, B. (1995) Curr. Top. Microbiol. Immunol., 199, 177-194. Im adenoviralen System kommt dem E2-late-Promotor zusammen mit dem E2-early-Promotor die Funktion zu, die adenovirale E2 Region bzw. die Gene E2A und E2B zu steuern. Dabei erfolgt zunächst die Synthese der E2 mRNA ausgehend vom E2-early-Promotor. Etwa fünf bis sieben Stunden nach Infektion einer Zelle erfolgt ein Umschalten auf den E2-late-Promotor. Der diesem Prozess zu Grunde liegende Mechanismus ist derzeit noch unbekannt.

In der frühen Phase der Infektion mit Adenoviren werden zunächst zwei mRNA-Produkte der E1A-Region hergestellt, die eine Größe von 13S bzw. 12S aufweisen. Untersuchungen haben ergeben, dass das E1A 12S-Protein die Aktivierung der E2-Region über den E2-late-Promotor verhindert bzw. reprimiert. Das Gen, das das E1A 13S-Protein kodiert, aktiviert dagegen die E2-Region bzw. Gene über den E2-early-Promotor. (Guilfoyle RA, Osheroff WP, Rossini, EMBO J 1985, 4, 707-713).

Weiterhin ist bekannt, dass eine Deletion des Bereiches der Nukleotide -51 bis - 33 des E2-late-Promotors die Synthese der E2 Region nahezu völlig reprimiert. (Guilfoyle RA, et al, aaO).

Es ist im Rahmen der vorliegenden Erfindung, dass jeglicher adenoviraler E2-late-Promotor verwendet werden kann. Derartige unterschiedliche adenovirale E2-late-Promotoren können durch die verschiedenen Formen der Adenoviren, wie sie im Stand der Technik bekannt sind, bedingt sein. Im Stand der Technik sind derzeit ca. 50 Subtypen bekannt, von denen prinzipiell ein jeder im Rahmen der vorliegenden Erfindung verwendet werden könnte, sei es als Vektor oder sei es als Quelle für einen E2-late-Promotor.

Der E2-late-Promotor weist eine Reihe von strukturellen und sequenziellen Merkmalen auf, die für seine Verwendung und insbesondere die Verwendung von Fragmenten des Promotors bedeutsam sein können. Ein derartiges Merkmal ist die Ausbildung einer Schleife (engl. "loop") im Bereich der Nukleotide -47 bis - 81, wobei mit der Position -1 direkt das erste Nukleotid bezeichnet wird, das unter der Kontrolle des Promotors transkribiert wird. Diese Schleife ist ein integraler Bestandteil der beiden hierin offenbarten Fragmente des E2-late-Promotors, die gleichwohl die hierin für den vollständigen E2-late-Promotor beschriebenen Eigenschaften aufweisen. Ein weiteres Merkmal, welches in den funktional aktiven Fragmenten des E2-late-Promotors bevorzugter Weise enthalten ist, ist die sogenannte Y-Box, die für die Bindung des YB-1-Proteins verantwortlich zu sein scheint. Weitere Elemente, die in bevorzugten Ausführungsformen des E2 late-Promotors und den erfindungsgemäßen Fragmenten enthalten sein können, sind die TATA-Box und die SPI-Bindungsstelle. Dabei ist die TATA-Box für die Initiation der Transkription von Bedeutung und ist gewöhnlicherweise etwa 25 bis 32 bp stromaufwärts von der Transkriptionsinitiationsstelle entfernt. Ein weiteres Merkmal des E2 late-Promotors bzw. eines funktional aktiven Fragmentes davon, das optional vorhanden sein kann, entweder einzeln oder in Ergänzung zu den anderen vorstehend beschriebenen Merkmalen, ist die sogenannte SPI-Bindungsstelle. Die SPI-Bindungsstelle wird durch die sogenannte GC-Box ausgebildet, an die der Transkriptionsfaktor SPI bindet. Pro Promotor kann mehr als eine GC-Box vorhanden sein.

Das hierin offenbarte Nukleinsäurekonstrukt bzw. der E2-late-Promotor oder ein funktional aktives Fragment davon, kann entweder in einer Form vorliegen, bei der YB-1 gebunden ist, oder in einer YB-1 -freien Form. Bei Bindung von YB-1 ist der Promotor funktional aktiv und es kann in einem geeigneten Transkriptionssystem eine Transkription erfolgen; bei Abwesenheit von YB-1 ist der Promotor nicht aktiv, so dass in einem Transkriptionssystem keine Transkription beobachtet werden kann. Geeignete Transkriptionssysteme sind beispielsweise beschrieben in Lewin, B., Gene: Lehrbuch der molekularen Genetik, VCH Verlagsgesellschaft, 6490 Weinheim, Germany.

Gemäß der vorliegenden Erfindung ist es möglich, dass praktisch eine jegliche Nukleinsäure unter die Kontrolle des adenoviralen E2-late-Promotors gestellt wird, der dann die Expression der Nukleinsäure steuert. Der E2-late-Promotor steht dabei unter der stringenten Kontrolle durch YB-1. Als mögliche Nukleinsäuren können dabei sowohl Gene wie auch allgemein kodierende Sequenzen, oder Fragmente davon, aber auch nicht-kodierende Nukleinsäuren verwendet werden. Bei der Expression von im weitesten Sinne kodierende Nukleinsäure und deren Steuerung durch den E2-late-Promotor ist dabei vorgesehen, dass die für Promotoren üblichen Erfordernisse gegeben sind, d. h. ein geeignetes Initiationscodon existiert und der Promotor in einem solchen Abstand von diesem Initiationscodon platziert wird, dass eine Translation möglich ist. Gleiches gilt grundsätzlich auch hinsichtlich der Erfordernisse, wie sie für die Transkription bestehen.

Grundsätzlich ist es im Rahmen der vorliegenden Erfindung, dass eine jegliche codierende Nukleinsäure verwendet werden kann. Mit Blick auf die spezifische Regulierbarkeit des Promotors durch YB-1 und damit der Anwendung des Vektors in einem biologischen System, das sich durch Abwesenheit oder Anwesenheit dieses Effektor auszeichnet, ergeben sich bevorzugte Kombinationen von codierenden Sequenzen mit dem E2-late-Promotor. Nachdem YB-1 insbesondere mit verschiedenen Tumorgeschehen in Verbindung gebracht wird, können bevorzugte Nukleinsäuren solche sein, die bei der Behandlung von Tumoren auf der molekularen Ebene bedeutsam sein können. Dazu gehören beispielsweise Apoptose-induzierende Gene. Mit dem Einschleusen von derartigen Gene in Tumorzellen, die YB-1 im Kern aufweisen (beispielsweise 30 % der Ovarialkarzinome [Kamura et al., 1999; Cancer, 85, 2450-2454; Shibao K, Takano H, Nakayama Y, Okazaki K, Nagata N, Izumi H, Uchiumi T, Kuwano M, Kohno K, Itoh H.Enhanced coexpression of YB-1 and DNA topoisomerase II alpha genes in human colorectal carcinomas.Int J Cancer. 1999 Dec 10;83(6):732-7]), sei es natürlicherweise oder induziert, können ausschließlich die Tumorzellen die an den Promotor gekoppelten Gene exprimieren mit der Folge, dass lediglich in diesen Zellen die durch die Apoptose-induzierenden Gene verursachte Apoptose abläuft. Ähnlich verhält es sich mit anderen, solchermaßen eingeschleusten Genen. Bevorzugterweise werden solche Gene eingeschleust, die zu einer Änderung des Verhaltens der Zellen, wie beispielsweise des Tumorcharakters der Zellen und/oder zur selektiven Abtötung der Tumorzellen führen. Neben den Apoptose-Genen können auch solche Gene eingeschleust werden, die sehr unmittelbar in das zelluläre Geschehen eingreifen, so z.B. Protease-Inhibitoren. Solche Protease-Inhibitoren sollen im allgemeinen das invasive Verhalten bzw die Metastisierung der Tumoren inhibieren. Darunter fallen Matrix-Metallo Proteasen, (MMP), Plasminogen Aktivator System (uPA), Cathepsine. Weiterhin kann der E2-late-Promotor erfindungsgemäß dazu verwendet werden, dass er die Expression viraler Proteine steuert, wobei die viralen Proteine solche sind, die normalerweise, d. h. in den natürlich vorkommenden Adenoviren, nicht unter der Kontrolle des E2-late-Promotors stehen. Insbesondere sind dies die viralen Proteine E3ADP, E4orf6 und E1B55k. E4orf6 ist ein multifunktionelles Protein, welches für die maximale virale DNA-Replikation und Partikelbildung benötigt wird. Ferner spielt es eine wichtige Rolle beim Splicing und Transport der viralen RNA. Zudem interagiert es mit dem viralen Protein E1B55k, um die Inaktivierung von p53 zu beschleunigen. E1B55k ist ebenfalls ein multifunktionelles Protein, welches in Wechselwirkung mit dem E4orf6-Protein den Export der viralen RNA fördert, wohingegen die zelleigenen RNAs im Zellkern zurückgehalten werden. Eine weitere wichtige Funktion von E1B55k besteht darin, alleine und/oder zusammen mit E4orf6 das zelluläre Protein p53 zu inaktivieren. E3ADP, auch als adenoviral death protein, d. h. adenovirales Todes-Protein, bezeichnet, ist ein integrales Membran-Glycoprotein, welches für eine effiziente Zellyse und Freisetzung der neu synthetisierten Viren benötigt wird. Die vorstehend genannten viralen Proteine sind ansonsten den Fachleuten auf dem Gebiet bekannt und in der Literatur beschrieben.

Die selektive Abtötung der Zellen kann entweder direkt unter dem Einfluss der eingeschleusten Gene oder indirekt infolge der durch die eingeschleusten Gene bedingten Änderungen in den Zellen erfolgen. Eine derartige Änderung kann beispielsweise auch dazu führen, dass weitere von außen zugeführte Verbindungen erst auf die Zellen wirken und somit zu beispielsweise einer Abtötung der Tumorzellen führen. Ein Beispiel für diesen Ansatz stellen Gene dar, die dem Pro-Drug-System zuzurechnen sind. Das Pro-Drug-System ist ein System von insbesondere Enzymen, die dazu führen, dass metabolisch nicht aktive chemische Verbindungen, die beispielsweise als Arzneimittel einem Organismus zugeführt werden, erst im Körper in die pharmazeutisch wirksame Form überführt werden. Ein Beispiel für ein Pro-Drug stellt beispielsweise das Thymidin-Kinase-System (TK-System) dar. Es beruht auf der Expression des Herplex Simplex Thymidin Kinase Gens (HSVtk) nach Zugabe der Prodrug Ganciclovir. Diese ist für den Menschen in dieser Form nicht toxisch. Die Thymidin-Kinase phosphoryliert das Substrat Ganciclovir. Es entsteht ein Purinanaloga, welches toxisch ist. Ein weiteres Beispiel stellt das System Cytosin Desaminase Gen (CD) dar.

Die Kopplung des adenoviralen E2-late-Promotors an eine nicht-kodierende Nukleinsäure ist beispielsweise auch an rRNA oder tRNA möglich. Insoweit ist eine verstärkte Expression dieser für das Funktionieren eines zellulären Systems wesentlichen RNA-Populationen möglich, die wiederum mit einer Vielzahl von zellulären Wirkungen und Funktionen verbunden sein können.

Eine weitere Form der nicht-kodierenden Nukleinsäuren, die unter die Kontrolle des adenoviralen E2-late-Promotors gestellt werden können, sind Aptamere, Ribozyme, anti-sense-Moleküle und siRNA. Aptamere sind dabei Nukleinsäuren, bevorzugterweise Ribonukleinsäuren, die spezifisch an ein Zielmolekül, gegen das sie selektiert wurden, binden. Die Herstellung derartiger Aptamere ist beispielsweise im europäischen Patent EP 0 533 838 beschrieben. Eine weitere Gruppe von Nukleinsäuren, die unter die Kontrolle des adenoviralen E2-late-Promotors gestellt werden können, sind anti-sense-Moleküle, deren Wirkprinzip darauf beruht, dass diese Moleküle mit mRNA einen Komplex bilden und somit die Translation der mRNA verhindern. In einer Ausführungsform sind anti-sense Moleküle auch in einer solchen Form bekannt, dass das zelluläre RNase H-System aktiviert und in Folge der enzymatischen Aktivität des RNase H-System die mit dem anti-sense Molekül komplexierte oder hybridisierte mRNA abgebaut wird.

Schließlich können die nicht-kodierenden Nukleinsäure auch Ribozyme sein, d. h. Nukleinsäuren, die katalytisch aktiv sind und entweder intramolekulare oder intermolekulare Nukleinsäuren, insbesondere Ribonukleinsäure, spalten, d.h. hydrolysieren können. In Folge der Sequenzspezifität von Ribozymen ist es möglich, spezifische Nukleinsäure-Populationen in einem biologischem System wie einer Zelle selektiv zu hydrolysieren und somit biologische Prozesse zu beeinflussen.

Das hierin offenbarte Nukleinsäurekonstrukt kann in dem Umfang ausgestaltet werden, wie dies vorstehend für die verschiedenen Verwendungen des E2-late-Promotors und Fragmenten davon beschrieben ist.

Das Nukleinsäurekonstrukt kann in verschiedenen Formen vorliegen. So ist es im Rahmen der vorliegenden Erfindung, dass das Nukleinsäurekonstrukt Teil eines Vektors ist. Derartige Vektoren sind den Fachleuten bekannt und umfassen beispielsweise Plasmide und Viren. Bevorzugter Weise sind die Vektoren solche für eukaryontische Zellen, insbesondere für Säugetierzellen. Virale Vektoren umfassen, unter anderem, adenovirale Vektoren, retrovirale Vektoren, adeno-assoziierte Vektoren (AAV) und Herpes-Simplex Vektoren. Alle Vektoren sind erwähnt bzw. beschrieben in Dougherty, GJ., Chaplin, D., Dougherty, ST., Chiu, RK, McBridge, Wh. In vivo gene therapy of cancer, Tumor Targeting, 2, 106-114 (1996); Advances in Pharmacology: Gene Therapy, Editor J. Thomas August, Volume 40, Academic Press.

Das erfindungsgemäße Nukleinsäurekonstrukt kann allgemein zur Herstellung von Medikamenten verwendet werden. Dabei bestehen hinsichtlich der Art der Indikationen für derartige Medikamente keine Einschränkungen, solange die Medikamente dadurch gekennzeichnet sind, dass sie entstehen oder zur Wirkung gelangen durch die Verwendung des adenoviralen E2-late-Promotors oder eines funktional aktiven Fragmentes davon, wie hierin offenbart. Mit anderen Worten, Medikamtente im Sinne der vorliegenden Erfindung sind auch solche, die aus bekannten Genen oder allgemein Nukleinsäuren bestehen, sofern diese unter der Kontrolle des adenoviralen late E2-Promotors oder eine funktional aktiven Fragmentes davon stehen.

Eine bevorzugte Indikation für die Anwendung der erfindungsgemäßen Medikamente stellen Tumorerkrankungen dar. Dies liegt in der hierin offenbarten Bindung von YB-1 an den adenoviralen E2-late-Promotor und der damit begründeten spezifischen Regulierbarkeit des Promotors begründet, so dass jene Nukleinsäure, die unter der Kontrolle des adenoviralen E2-late-Promotors oder eines funktional aktiven Fragmentes davon steht, spezifisch in Tumorzellen exprimiert wird, die YB-1 im Kern aufweisen. Normale, insbesondere humane, Zellen, weisen YB-1 nur im Zytoplasma auf, so dass diese keine Expression der unter der Kontrolle des adenoviralen E2-late-Promotors oder eines funktional aktiven Fragmentes davon stehenden Nukleinsäure zeigen.

Infolge der Kopplung der Aktivierung der unter der Kontrolle des adenoviralen E2-late-Promotors oder eines funktional aktiven Fragmentes davon stehenden Nukleinsäure mit im Zellkern vorhandene YB-1 können mit dem erfindungsgemäßen Nukleinsäurekonstrukt auch solche Erkrankungen und insbesondere Tumorerkrankungen behandelt werden, bei denen YB-1 nur dann im Zellkern vorliegt, wenn bestimmte Bedingungen vorliegen, die dazu führen, dass YB-1 im Kern ausschließlich, überwiegend oder in einem gegenüber dem Nicht-Vorliegen der besagten bestimmten Bedingungen verstärkten Umfang vorliegt. Im Bereich der Tumorerkrankungen kann die Lokalisation von YB-1 im Kern dadurch bewirkt werden, dass die Zellen Stressfaktoren ausgesetzt werden. Derartige Stressfaktoren sind beispielsweise Hyperthermie, UV-Strahlung oder die Behandlung der Zellen bzw. des diese enthaltenden Organismus durch Zytostatika. Derartige Zytostatika umfassen, unter anderem, cis-Platin.

Weitere Zellen, die der Behandlung durch das erfindungsgemäße Nukleinsäurekonstrukt grundsätzlich zugänglich sind, sind die sogenannten vielfachresistenten Tumorzellen. Die Vielfachresistenz (engl. multi drug resistance) wird durch die Synthese des P-Glycoproteins bedingt. Der Zusammenhang zwischen YB-1 und der MDR-1-Genexpression (MDR - multiple drug resistance) wurde beschrieben durch Bargou et al. (Bargou, R.C. et al., Nature Med. 3, 1997, 4 : 447 - 450). Infolge dieses Zusammenhangs sind auch solche Tumorzellen und diese enthaltenden Tumoren mittels des erfindungsgemäßen Nukleinsäurekonstruktes adressierbar und therapierbar, die P-Glycoprotein positiv sind.

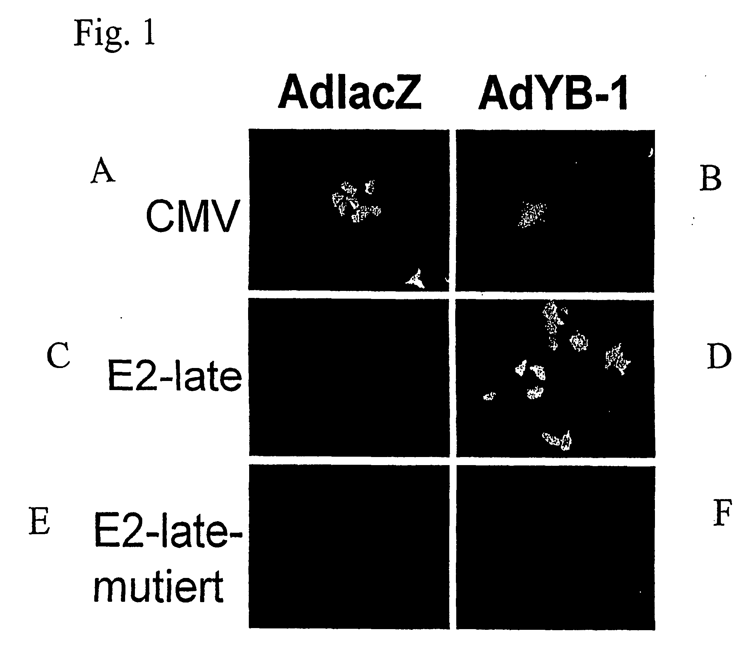

Die Erfindung wird im folgenden anhand der Figuren, Beispiele und des Sequenzprotokolls veranschaulicht, aus denen sich weitere Merkmale, Ausführungsformen und Vorteilen der Erfindung ergeben. Dabei zeigt
- Fig. 1: fluoreszenzmikroskopische Aufnahmen von U20S-Zellen, die mit einem Vektor kodierend für Grün-fluoreszierendes Protein unter Kontrolle des CMV-Promotors (Fig. 1A, 1B), unter Kontrolle des E2-late-Promotors (Fig. 1C, 1D) und unter Kontrolle eines in der YB-1-Box mutierten E2-late-Promotors (Fig. 1E, 1F) transfiziert wurden, nach Infektion mit einem E1/E3 deletierten Adenovirus (Fig. 1A, 1C, 1E) und einen YB-1 exprimierenden E1/E3-deletierten Adenovirus (Fig. 1B, 1D, 1F); und

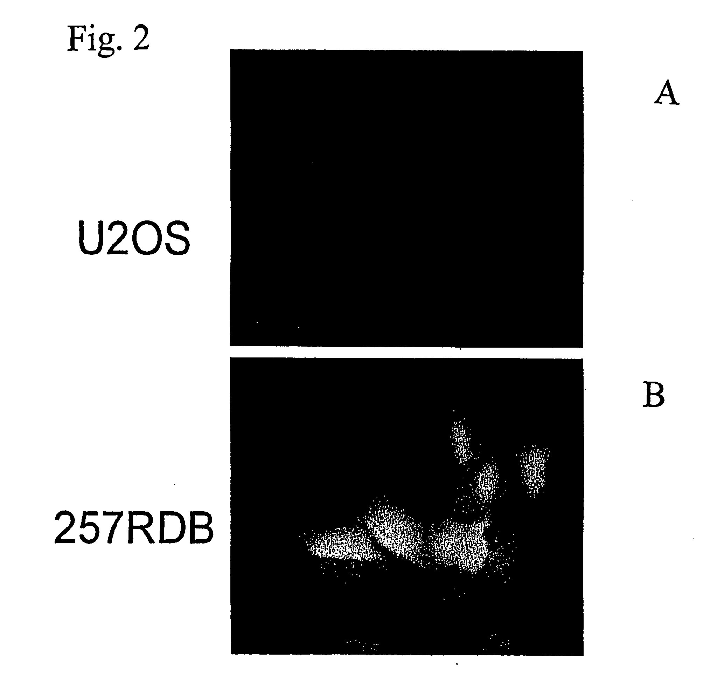

- Fig. 2: fluoreszenzmikroskopische Aufnahmen von YB-1-kernnegativen U2OS-Zellen (Fig. 2A) und YB-1-kempositiven Zellen (Fig. 2B) nach Transfektion mit einem Plasmid, das für Grün-fluoreszierendes Protein unter der Kontrolle des Adenoviralen E2-late-Promotors kodiert.

### Beispiele

### Beispiel 1: Der E2-late-Promotor ist YB-1-spezifisch

Der E2-late-Promotor wurden in den Vektor pGL3-Enhancer (Firma Promega) in die XhoI- und HindIII-Schnittstelle kloniert. Als Reportergen besitzt dieser Vektor das Luciferase-Gen oder alternativ das GFP-Gen. Sobald GFP exprimiert wird, leuchten die Zellen grün auf. Zunächst wurden 200.000 U2OS Zellen pro Napf in 6-Napf-Platten ausgelegt. Nach 24 Stunden erfolgte die Transfektion der die verschiedenen Fragmente des E2-late-Promotors enthaltenden verschiedenen Vektoren mittels Superfect nach Herstellerangaben (Qiagen). Die Plasmide wurden aus dem pGL3-Enhancer-Vektor, Firma Promega, hergestellt, wobei das Luziferasegen durch das Reportergen GFP (Grün-fluoreszierendes Protein) ausgetauscht.

Nach weiteren 24 Stunden wurden die Zellen mit 50 pfu/Zelle mit einem E1/E3 deletierten Adenovirus (AdlacZ, linke Spalte von Fig. 1) und AdYB-1 (rechte Spalte von Fig. 1) infiziert. AdYB-1 ist ein E1/E3 deletiertes Adenovirus, welches als Transgen den Transkriptionsfaktor YB-1 exprimiert. Nach weiteren 24 Stunden erfolgte die Auswertung unter einem Fluoreszenzmikroskop. Das Ergebnis zeigt deutlich, dass durch die Expression von YB-1 nur der intakte E2-late-Promotor eingeschaltet wird, wie in Fig. 1D dargestellt. Die in Fig. 1A und 1B dargestellten Zellen wurden als Positivkontrolle mit Plasmidkonstrukten transfiziert, bei denen das Grün-fluoreszierende Protein unter die Kontrolle des Cytomegalovirus-Promotors (CMV) gestellt wurde. Bei den in Fig. 1C und Fig. 1D dargestellten Konstrukten bzw. Versuchen wurde der E2-late-Promotor erfindungsgemäß verwendet, d. h. die Expression des Grünfluoreszierenden Proteins unter die Kontrolle dieses Promotors gestellt. Bei den in Fig. 1E und Fig. 1F dargestellten Zellen wurde anstelle des E2-late-Promotors von Adenovirus ein mutierter E2-late-Promotor von Adenovirus verwendet. Die Mutation lag dabei in der YB-1-Box, wobei die Sequenz GCCTG statt ATTGG verwendet wurde.

### Beispiel 2: E2-late-Promotor ist spezifisch für YB-1-kernpositive Zellen

Für die Untersuchung der Spezifität des E2-late-Promotors in resistenten YB-1-kernpositiven Zellen wurde das folgende Experiment durchgeführt.

Es wurden 200.000 Zellen pro Napf in einer 6-Napf-Platte ausgelegt. Ein synthetisierter E2-late-Promotor im pGL3-Enhancer-Vektor (erhältlich von Promega) mit dem Reportergen GFP wurde mittels Superfect der Firma Qiagen nach Herstellerangaben 24 Stunden später in die Zellen tranfiziert. Nach weiteren 48 Stunden erfolgte die Auswertung unter einem Fluoreszenzmikroskop.

Fig. 2A zeigt das Ergebnis der Osteosarkomzellen U2OS, die kein YB-1 im Kern aufweisen. In Fig. 2B ist das Ergebnis unter Verwendung von gegen eine Vielzahl von Wirkstoffen resistenten (engl. multi drug resistant) Magenkarzinomzellen 257RDB dargestellt. Bei diesen Zellen ist YB-1 im Zellkern lokalisiert. Das Ergebnis zeigt deutlich, dass nur in YB-1 kernpositiven 257RDB-Zellen das Reportergen GFP über den E2-late Promotor eingeschaltet wird.

## Patentansprüche

1. Verwendung eines adenoviralen E2-late-Promotors oder eines funktional aktiven Fragments davon zur durch YB-1 kontrollierten Expression von Genen, die verschieden sind von den durch den E2-late-Promotor gesteuerten adenoviralen Genen oder Nukleinsäuren, zur Herstellung eines Medikamentes, wobei der Promotor und/oder das Promotor-Fragment eine Bindungsstelle für YB-1 aufweist.

2. In vitro-Verwendung eines adenoviralen E2-late-Promotors oder eines funktional aktiven Fragments davon zur durch YB-1 kontrollierten Expression von Genen, die verschieden sind von den durch den E2-late-Promotor gesteuerten adenoviralen Genen oder Nukleinsäuren, wobei der Promotor und/oder das Promotor-Fragment eine Bindungsstelle für YB-1 aufweist.

3. Verwendung eines adenoviralen E2-late-Promotors oder eines funktional aktiven Fragments davon zur YB-1 kontrollierten Expression eines Transgens oder einer transgenen Nukleinsäure zur Herstellung eines Medikamentes, wobei der Promotor und/oder das Promotor-Fragment eine Bindungsstelle für YB-1 aufweist.

4. In vitro-Verwendung eines adenoviralen E2-late-Promotors oder eines funktional aktiven Fragments davon zur YB-1 kontrollierten Expression eines Transgens oder einer transgenen Nukleinsäure, wobei der Promotor und/oder das Promotor-Fragment eine Bindungsstelle für YB-1 aufweist.

5. Verwendung nach einem der Ansprüche 1 bis 4, **dadurch gekennzeichnet, dass** das Promotor-Fragment eine Sequenz gemäß SEQ. ID. No. 1 umfasst.

6. Verwendung nach einem der Ansprüche 1 bis 4 , **dadurch gekennzeichnet, dass** das Promotor-Fragment eine Sequenz gemäß SEQ. ID. No. 2 aufweist.

7. Verwendung nach einem der Ansprüche 1 bis 6, **dadurch gekennzeichnet, dass** der Promotor und/oder das Fragment mindestens ein Element aufweist, das ausgewählt ist aus der Gruppe, die die Y-Box, die TATA-Box und die SPI-Bindungsstelle umfasst.

8. Verwendung nach einem der Ansprüche 1 bis 7, **dadurch gekennzeichnet, dass** der Promotor und/oder das Promotor-Fragment YB-1 gebunden aufweist.

9. Verwendung nach einem der Ansprüche 1 bis 8, **dadurch gekennzeichnet, dass** das Transgen und/oder das von dem durch den E2-late-Promotor gesteuerte Gen und/oder Nukleinsäure ausgewählt ist aus der Gruppe von Genen, die Apoptose-induzierende Gene, Gene für Pro-Drug-Systeme und Gene für Protease-Inhibitoren umfasst.

10. Verwendung nach einem der Ansprüche 1 bis 9, **dadurch gekennzeichnet, dass** das Transgen oder die von der durch den adenoviralen E2-late-Promotor gesteuerten Gene oder Nukleinsäure(n) ausgewählt ist/sind aus der Gruppe, die Antisense-Moleküle, Ribozyme, Aptamere und siRNA umfasst.

11. Nukleinsäurekonstrukt zur Verwendung als Medikament umfassend einen durch YB-1 kontrollierten adenoviralen E2-late-Promotor oder ein funktional aktives Fragment davon und eine Nukleinsäure, wobei die Nukleinsäure ausgewählt ist aus der Gruppe, die Transgene, Gene und Nukleinsäuren, die jeweils verschieden sind von den durch einen E2-late-Promotor gesteuerten adenoviralen Nukleinsäuren, umfasst, wobei der Promotor und/oder das Promotor-Fragment eine Bindungsstelle für YB-1 aufweist.

12. Nukleinsäurekonstrukt nach Anspruch 11, **dadurch gekennzeichnet, dass** das Promotorfragment eine Nukleinsäuresequenz umfasst, die ausgewählt ist aus der Gruppe, die SEQ ID No.1 und SEQ ID No.2 umfasst.

13. Vektor umfassend ein Nukleinsäurekonstrukt nach Anspruch 11 oder 12 zur Verwendung als Medikament.

14. Verwendung eines Nukleinsäurekonstruktes zur Herstellung eines Medikaments, wobei das Nukleinsäurekonstrukt einen durch YB-1 kontrollierten adenoviralen E2-late-Promotor oder ein funktional aktives Fragment davon und eine Nukleinsäure umfasst, wobei die Nukleinsäure ausgewählt ist aus der Gruppe, die Transgene, Gene oder Nukleinsäuren, die jeweils verschieden sind von den durch einen E2-late-Promotor gesteuerten adenoviralen Nukleinsäure, umfasst, wobei der Promotor und/oder das Promotor-Fragment eine Bindungsstelle für YB-1 aufweist.

15. Verwendung nach Anspruch 14, **dadurch gekennzeichnet, dass** das Promotorfragment eine Nukleinsäure umfasst, die ausgewählt ist aus der Gruppe, die SEQ. ID. No. 1 und SEQ. ID. No. 2 umfasst.

16. Verwendung nach Anspruch 14 oder 15, **dadurch gekennzeichnet, dass** das Medikament zur Behandlung von Tumoren verwendet wird.

17. Verwendung nach Anspruch 16, **dadurch gekennzeichnet, dass** die Tumoren solche sind, die YB-1 im Kern aufweisen.

18. Verwendung nach einem der Ansprüche 14 bis 17, **dadurch gekennzeichnet, dass** die Tumoren solche sind, die YB-1 im Kern aufweisen, bevorzugter Weise YB-1 bei Vorliegen eines Stressfaktors im Zellkern aufweisen.

19. Verwendung nach Anspruch 18, **dadurch gekennzeichnet, dass** der Stressfaktor ausgewählt ist aus der Gruppe, die Hyperthermie, UV-Exposition und Exposition gegenüber Zytostatika umfasst.

20. Verwendung nach einem der Ansprüche 14 bis 19, **dadurch gekennzeichnet, dass** das Medikament zusammen mit Zytostatika und/oder Hyperthermie verwendet wird.

21. Verwendung nach einem der Ansprüche 14 bis 20, **dadurch gekennzeichnet, dass** der Tumor Tumorzellen mit Vielfachresistenzen aufweist.

## Claims

1. Use of an adenoviral E2-late promoter or a functionally active fragment thereof for YB-1 controlled expression of genes which are different from the adenoviral genes or nucleic acids controlled by the E2-late promoter, for the manufacture of a medicament, whereby the promoter and/or the promoter fragment comprises a YB-1 binding site.

2. In vitro use of an adenoviral E2-late promoter or a functionally active fragment thereof for YB-1 controlled expression of genes which are different from the adenoviral genes or nucleic acids controlled by the E2-late promoter, whereby the promoter and/or the promoter fragment comprises a YB-1 binding site.

3. Use of an adenoviral E2-late promoter or a functionally active fragment thereof for YB-1 controlled expression of a transgene or a transgene nucleic acid for the manufacture of a medicament, whereby the promoter and/or the promoter fragment comprises a YB-1 binding site.

4. In vitro use of an adenoviral E2-late promoter or a functionally active fragment thereof for YB-1 controlled expression of a transgene or a transgene nucleic acid, whereby the promoter and/or the promoter fragment comprises a YB-1 binding site.

5. Use according to any one of claims 1 to 4, **characterized in that** the promoter fragment comprises a sequence according to SEQ. ID. No. 1.

6. Use according to any one of claims 1 to 4, **characterized in that** the promoter fragment comprises a sequence according to SEQ.ID. No. 2.

7. Use according to any one of claims 1 to 6, **characterized in that** the promoter and/or the promoter fragment comprises at least one element selected from the group comprising the Y-Box, the TATA-Box and the SPI-binding site.

8. Use according to any one of claims 1 to 7, **characterized in that** the promoter and/or the promoter fragment has YB-1 bound to it.

9. Use according to any one of claims 1 to 8, **characterized in that** the transgene and/or the gene and/or the nucleic acid controlled by the E2-late promoter is selected from the group comprising apoptosis inducing genes, genes for pro-drug-systems and genes for protease inhibitors.

10. Use according to any one of claims 1 to 9, **characterized in that** the transgene and/or the genes or the nucleic acid(s) controlled by the adenoviral E2-late promoter is/are selected from the group comprising antisense molecules, ribozymes, aptamers and siRNA.

11. Nucleic acid construct for use as a medicament, comprising an adenoviral E2-late promoter controlled by YB-1 or a functionally active fragment thereof, and a nucleic acid, whereby the nucleic acid is selected from the group comprising transgenes, genes and nucleic acids which are each different from the adenoviral nucleic acids controlled by the E2-late promoter, whereby the promoter and/or the promoter fragment comprises a binding site for YB-1.

12. Nucleic acid construct according to claim 11, **characterized in that** the promoter fragment comprises a nucleic acid sequence selected from the group comprising SEQ. ID. No. 1 and SEQ. ID. No. 2.

13. Vector comprising a nucleic acid construct according to claim 11 or 12, for use as a medicament.

14. Use of a nucleic acid construct for the manufacture of a medicament, whereby the nucleic acid construct comprises a YB-1 control adenoviral E2-late promoter or a functionally active fragment thereof, and a nucleic acid, whereby the nucleic acid is selected from the group comprising transgenes, genes or nucleic acids which are each different from the adenoviral nucleic acid controlled by the E2-late promoter, whereby the promoter and/or the promoter fragment comprises a YB-1 binding site.

15. Use according to claim 14, **characterized in that** the promoter fragment comprises a nucleic acid selected from the group comprising SEQ. ID. No. 1 and SEQ. ID. No. 2.

16. Use according to claim 14 or 15, **characterized in that** the medicament is used for the treatment of tumors.

17. Use according to claim 16, **characterized in that** the tumors are those having YB-1 in the nucleus.

18. Use according to any one of claims 14 to 17, **characterized in that** the tumors are those having YB-1 in the nucleus, preferably those having YB-1 in the cellular nucleus in the presence of a stress factor.

19. Use according to claim 18, **characterized in that** the stress factor is selected from the group comprising hyperthermia, UV exposition and exposition against cytostatics.

20. Use according to any one of claims 14 to 19, **characterized in that** the medicament is used together with cytostatics and/or hyperthermia.

21. Use according to any one of claims 14 to 20, **characterized in that** the tumor comprises tumor cells being multiple resistant.

## Revendications

1. Utilisation d'un promoteur tardif E2 adénoviral ou d'un fragment fonctionnellement actif de ce dernier pour l'expression, contrôlée par YB-1, de gènes qui sont différents des gènes adénoviraux ou d'acides nucléiques contrôlés par le promoteur tardif E2, pour la fabrication d'un médicament, le promoteur et/ou le fragment du promoteur comprenant un site de liaison à YB-1.

2. Utilisation in vitro d'un promoteur tardif E2 adénoviral ou d'un fragment fonctionnellement actif de ce dernier, pour l'expression, contrôlée par YB-1, de gènes qui sont différents de gènes adénoviraux ou d'acides nucléiques contrôlés par le promoteur tardif E2, le promoteur et/ou le fragment de promoteur comprenant un site de liaison à YB-1.

3. Utilisation d'un promoteur tardif E2 adénoviral ou d'un fragment fonctionnellement actif de ce dernier pour l'expression, contrôlée par YB-1, d'un transgène ou d'un acide nucléique de transgène, pour la fabrication d'un médicament, le promoteur et/ou le fragment de promoteur comprenant un site de liaison à YB-1.

4. Utilisation in vitro d'un promoteur tardif E2 adénoviral ou d'un fragment fonctionnellement actif de ce dernier, pour l'expression, contrôlée par YB-1, d'un transgène ou d'un acide nucléique de transgène, le promoteur et/ou le fragment de promoteur comprenant un site de liaison à YB-1.

5. Utilisation selon l'une quelconque des revendications 1 à 4, **caractérisée en ce que** le fragment de promoteur comprend une séquence selon SEQ ID N°1.

6. Utilisation selon l'une quelconque des revendications 1 à 4, **caractérisée en ce que** le fragment de promoteur comprend une séquence selon SEQ ID N°2.

7. Utilisation selon l'une quelconque des revendications 1 à 6, **caractérisée en ce que** le promoteur et/ou le fragment de promoteur comprend au moins un élément choisi dans le groupe comprenant la boîte Y, la boîte TATA et le site de liaison à SPI.

8. Utilisation selon l'une quelconque des revendications 1 à 7, **caractérisée en ce que** YB-1 est lié au promoteur et/ou au fragment de promoteur.

9. Utilisation selon l'une quelconque des revendications 1 à 8, **caractérisée en ce que** le transgène et/ou le gène et/ou l'acide nucléique contrôlé par le promoteur tardif E2 est choisi dans le groupe comprenant les gènes d'induction de l'apoptose, les gènes de systèmes de promédicament et les gènes d'inhibiteurs de protéases.

10. Utilisation selon l'une quelconque des revendications 1 à 9, **caractérisée en ce que** le transgène et/ou les gènes ou l'/les acide(s) nucléique(s) contrôlé(s) par le promoteur tardif E2 adénoviral est/sont choisi(s) dans le groupe comprenant les molécules antisens, les ribozymes, les aptamères et l'ARNsi.

11. Construction d'acide nucléique pour son utilisation en tant que médicament, comprenant un promoteur tardif E2 adénoviral contrôlé par YB-1, ou un fragment fonctionnellement actif de ce promoteur, et un acide nucléique, dans laquelle l'acide nucléique est choisi dans le groupe comprenant les transgènes, les gènes et les acides nucléiques, qui sont chacun différents des acides nucléiques adénoviraux contrôlés par le promoteur tardif E2, le promoteur et/ou le fragment de promoteur comprenant un site de liaison à YB-1.

12. Construction d'acide nucléique selon la revendication 11, **caractérisée en ce que** le fragment de promoteur comprend une séquence d'acide nucléique choisie dans le groupe comprenant SEQ ID N°1 et SEQ ID N°2.

13. Vecteur comprenant une construction d'acide nucléique selon la revendication 11 ou 12 pour son utilisation en tant que médicament.

14. Utilisation d'une construction d'acide nucléique pour la fabrication d'un médicament, dans laquelle la construction d'acide nucléique comprend un promoteur tardif E2 adénoviral de contrôle de l'YB-1 ou un fragment fonctionnellement actif de ce promoteur, et un acide nucléique, dans laquelle l'acide nucléique est choisi dans le groupe comprenant les transgènes, les gènes ou les acides nucléiques qui sont chacun différents de l'acide nucléique adénoviral contrôlé par le promoteur tardif E2, dans laquelle le promoteur et/ou le fragment de promoteur comprend un site de liaison à YB-1.

15. Utilisation selon la revendication 14, **caractérisée en ce que** le fragment de promoteur comprend un acide nucléique choisi dans le groupe comprenant SEQ ID N°1 et SEQ ID N°2.

16. Utilisation selon la revendication 14 ou 15, **caractérisée en ce que** le médicament est utilisé pour le traitement de tumeurs.

17. Utilisation selon la revendication 16, **caractérisée en ce que** les tumeurs sont celles qui ont un YB-1 dans le noyau.

18. Utilisation selon l'une quelconque des revendications 14 à 17, **caractérisée en ce que** les tumeurs sont celles qui ont un YB-1 dans le noyau, de préférence celles qui ont un YB-1 dans le noyau cellulaire en présence d'un facteur de stress.

19. Utilisation selon la revendication 18, **caractérisée en ce que** le facteur de stress est choisi dans le groupe comprenant une hyperthermie, une exposition à des UV et une exposition à des cytostatiques.

20. Utilisation selon l'une quelconque des revendications 14 à 19, **caractérisée en ce que** le médicament est utilisé en même temps que des cytostatiques et/ou une hyperthermie.

21. Utilisation selon l'une quelconque des revendications 14 à 20, **caractérisée en ce que** la tumeur comprend des cellules tumorales multirésistantes.
